# EUROPEAN PATENT SPECIFICATION

(11) **EP 3 792 275 B1**
(45) Date of publication and mention of the grant of the patent: **10.04.2024**
(21) Application number: 19197142.3
(22) Date of filing: 13.09.2019
(51) Int. Cl.: C07K 14/47, A61K 38/17

(54) **METHOD OF ISOLATING MUCIN FROM MUCIN CONTAINING TISSUE**
VERFAHREN ZUR ISOLIERUNG VON MUCIN AUS MUCINHALTIGEM GEWEBE
PROCÉDÉ D'ISOLATION DE MUCINE DANS DES TISSUS CONTENANT DES MUCINES

(43) Date of publication of application: 17.03.2021
(73) Proprietor: Technische Universität München, 80333 München (DE)
(72) Inventor: LIELEG, Oliver, 85748 Garching (DE); WINKELJANN, Benjamin, 86899 Landsberg am Lech (DE); MARCZYNSKI, Matthias, 80331 Muenchen (DE); WEBER-HOHENGRUND, Max, 93161 Sinzing (DE)
(74) Representative: Schiweck Weinzierl Koch Patentanwälte Partnerschaft mbB

(56) References cited:
- EP-A1- 0 363 646
- EP-A1- 1 947 111
- EP-B1- 1 947 111
- WO-A1-81/02977
- SCHOMIG ET AL.: "An optimized purification process for porcine gastric mucin (PGM) with preservation of its native functional properties", RSC ADVANCES, vol. 6, no. 50, 2016, pages 44932-44943, XP055655417,
- KANTARCI N ET AL: "Bubble column reactors", PROCESS BIOCHEMISTRY, ELSEVIER LTD, GB, vol. 40, no. 7, 1 June 2005 (2005-06-01), pages 2263-2283, XP027794182, ISSN: 1359-5113 [retrieved on 2005-06-01]

## Description

### FIELD OF THE INVENTION

The present invention relates to a method of isolating mucin from a mucin containing tissue. In addition, the present disclosure describes mucin obtained by this method as well as a composition comprising the mucin obtained by the method. The present invention further relates to a use of a bubble column for isolating mucin from mucin containing tissue.

### BACKGROUND OF THE INVENTION

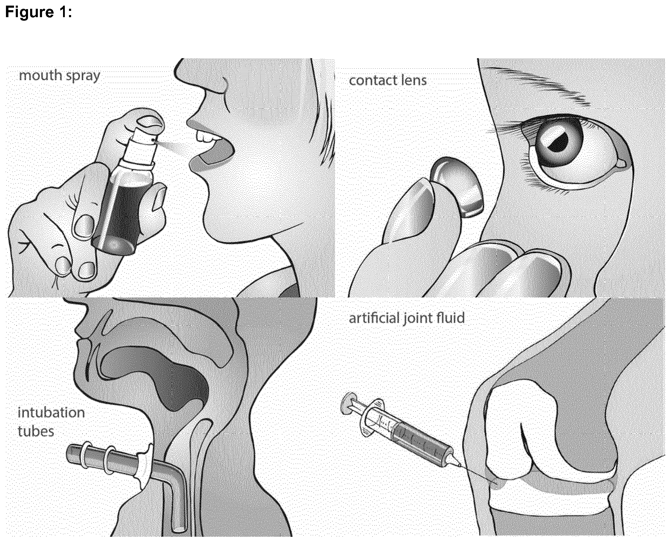

Mucins are highly glycosylated proteins, which are found in mucous membranes. For the mucous covered tissue, the ability of mucin to form a gel serves as a barrier against invading germs and as biological lubricants. Thus, mucins enable e.g. the painless chewing and swallowing of food or the blinking of the eyelid. These properties make mucin interesting for various medical applications, e.g. as lubricious ingredient in eye drops or mouth sprays or as germ protective lubricative for coatings of medical devices such as catheters, intubation tubes, contact lenses **(****Figure 1****).** Injected mucin can, for example, also be used as a lubricious, artificial joint fluid in therapy for osteoarthritis **(****Figure 1****).**

There are currently about 20 different types of mucin known to be found in the human body. However, in research typically mucins from vertebrates such as pigs and cattle are used, since the structure of porcine and bovine mucins is extremely similar to human mucins. Generally, mucins consist of a long polypeptide backbone of several thousand amino acids. Attached to these amino acids are various sugar chains, which contain negatively charged motifs such as sialic acids and/or sulfates, and which form the central area of the mucin molecule.

The mucins currently available from commercial sources have been shown to have lost some lubricious properties. Furthermore, they cannot be detected by commercially available antibodies. This is assumed to be due to an aggressive purification damaging the structure of the glycoproteins. To avoid structural damage and to preserve the mucin's lubricious properties, respectively, manual purification is currently the medium of choice.

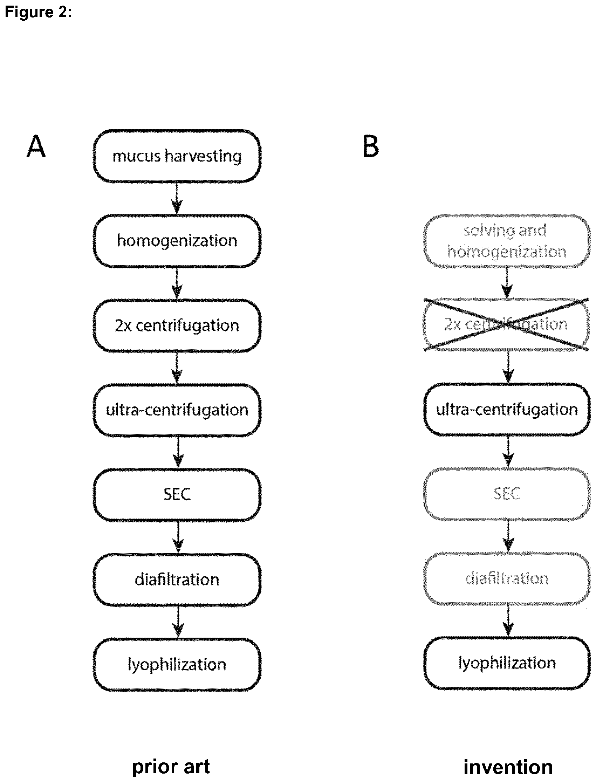

The state of the art method for manual mucin isolation is described in Schömig et al., An optimized purification process for porcine gastric mucin (PGM) with preservation of its native functional properties, RSC Advances 6 (50) 44932-44943 (2016). As shown in **Figure 2A****,** this method can be divided into several steps and begins with the mucus harvesting. For this step, a porcine stomach must first be opened with scissors to remove remaining food residues. Then, the inside of the stomach is rinsed under running water since the food residues are often quite stubbornly stuck to the stomach wall. The stomach is then put on ice and the mucus is removed by scraping the inner stomach wall with a spoon. Since the inner wall of the stomach is not smooth, but rather has strong furrows, it is relatively difficult and time-consuming to remove the mucus completely. Thus, the mucus harvesting can take about a quarter of an hour of manual work per stomach. Further, the harvesting process highly depends on the skill of the person performing the mucus harvesting, since the rinsing and scraping off of the stomach wall can result in a loss of mucus and thus in a loss of mucin. After the harvesting, the mucus is collected in a glass and pooled with the mucus of several other porcine stomachs. The pooled mucus is then diluted 1:5 with phosphate buffered saline (PBS), whereby the pH of the acid environment of the stomach (approx. pH=2 to 4) is raised to a neutral value. Doing so, the neutral pH reduces the activity of the protease pepsin, which degrades mucin. The mucin solution is then homogenized with a magnetic stirrer at approx. 4°C overnight. Afterwards, the mucin solution is subjected to a centrifugation at 8300 × g for 30 min to separate and thus remove food residues, cell residues and other impurities. The supernatant of the centrifuged mucin solution is then centrifuged at 15000 × g for 45 min before it is subjected to an ultracentrifugation at 150000 x g for 60 min to further remove impurities. After a concentration step using cross-flow ultrafiltration, the mucin containing fraction is separated from smaller proteins in a size exclusion chromatography (SEC). Thereafter, the salt content of the mucin solution is reduced to a conductivity of <100µS/cm by diafiltration using distilled water. The mucin is then lyophilized for long-term storage.

As evident from the above, the manual mucin isolation of Schömig et al., *supra,* contains many steps, making the isolation time-consuming and requiring complicated logistics. There is thus a need for the provision of a simpler method for mucin isolation.

Accordingly, it is an object of the invention to provide a method of isolating mucin from a mucin containing tissue that avoids these drawbacks.

### SUMMARY OF THE INVENTION

This object is accomplished by the method, the mucin and the use of an aeriated bubble column having the features of the independent claims.

The invention provides a method of isolating mucin from a mucin containing tissue comprising contacting the mucin containing tissue with an aqueous solution in an aeriated bubble column under conditions suitable for transferring mucin into the aqueous solution, thereby producing a mucin containing solution.

The present disclosure also provides a mucin obtained by the method of the invention, said disclosure is not part of the invention. Moreover and also not part of the invention, the herein described disclosure provides a composition comprising the mucin obtained by the method of the invention.

Further, the invention provides a use of a bubble column for isolating mucin from mucin containing tissue.

The claimed invention is defined in the appended claims.

### BRIEF DESCRIPTION OF THE DRAWINGS

The invention will be better understood with reference to the detailed description when considered in conjunction with the non-limiting examples and the drawings, in which:
**Figure 1** shows examples of medical applications in which the pathogen protective and lubricious properties of mucin can be used for. Mucin can, for example, be used as a lubricious ingredient in eye drops or mouth sprays. Further, mucin can be used as pathogen protective lubricative for coatings of medical advices such as contact lenses or intubation tubes. Mucin could also be injected into a joint as an artificial joint fluid and thus be used in therapy for osteoarthritis;
**Figure 2** shows the comparison of the experimental steps of a manual mucin isolation according to the prior art with an illustrative embodiment of the method of mucin isolation according to the present invention; **Figure 2** **A** shows the known PGM purification of Schömig et al., *supra.* **Figure 2** **B** shows an embodiment of the present invention. In this method illustrated by **Fig. 2B****,** the mucus is separated from the surface of the stomach during incubation in a bubble column. Once the solid and liquid phases have been separated, a single ultracentrifugation is sufficient to remove remaining impurities. Afterwards, a SEC is performed with a buffer that has a higher salt concentration in comparison to the buffer used in the SEC step of the prior art. This is followed by a subsequent washing step with a salt solution before a desalination is performed to set the conductivity to <10µS/cm. Finally, the mucin is dried e.g. via lyophilization;

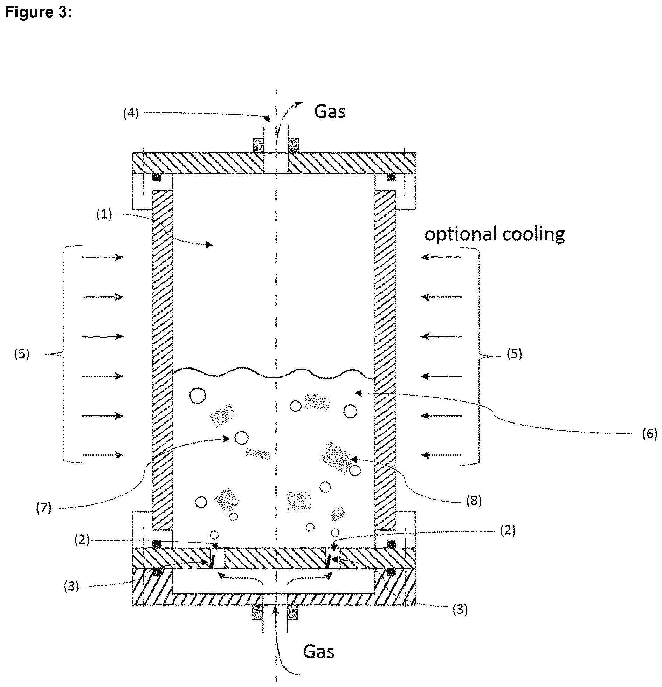

**Figure 3** shows a schematic configuration of an exemplary bubble column suitable for carrying out a method of mucin isolation of the present invention. The bubble column shown in **Figure 3** comprises a hollow chamber (1) which is sealed to the environment, an air inlet comprising of several nozzles (2) at the bottom, a non-return valve (3) ensuring that liquid cannot leak, an air outlet (4) mounted on the top and optionally a filter and a return system to prevent foam from leaking and to return the liquid to the system (not shown). The chamber (1) may optionally be equipped with an external cooling system schematically depicted by the reference arrows (5). Additionally, the chamber (1) may optionally be equipped with a control system for pH and conductivity of the liquid. Further, the chamber including all attachments may be completely demountable for easy cleaning and sterilization. The chamber (1) may also be filled with an aqueous solution (6), which is aeriated by a gas whereby bubbles (7) are generated. These bubbles (7) mix the aqueous solution and the pieces of the mucin containing tissue (8) contained therein;

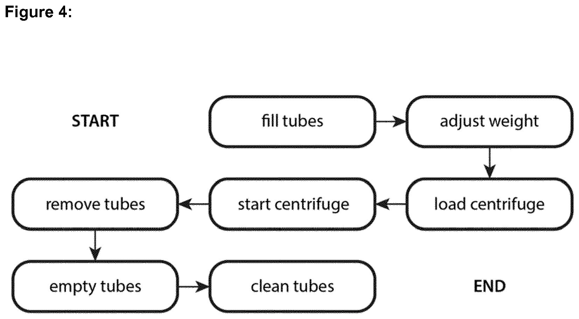

**Figure 4** shows the following manual steps during centrifugation following the protocol Schömig et al., *supra,* which are spared by the method of the present invention:
   - the filling of the tubes;
   - the taring of the filled tubes;
   - the loading of the centrifuge;
   - the starting of the centrifuge;
   - the removing of the tubes;
   - the emptying of the tubes; and
   - the cleaning of the tubes.

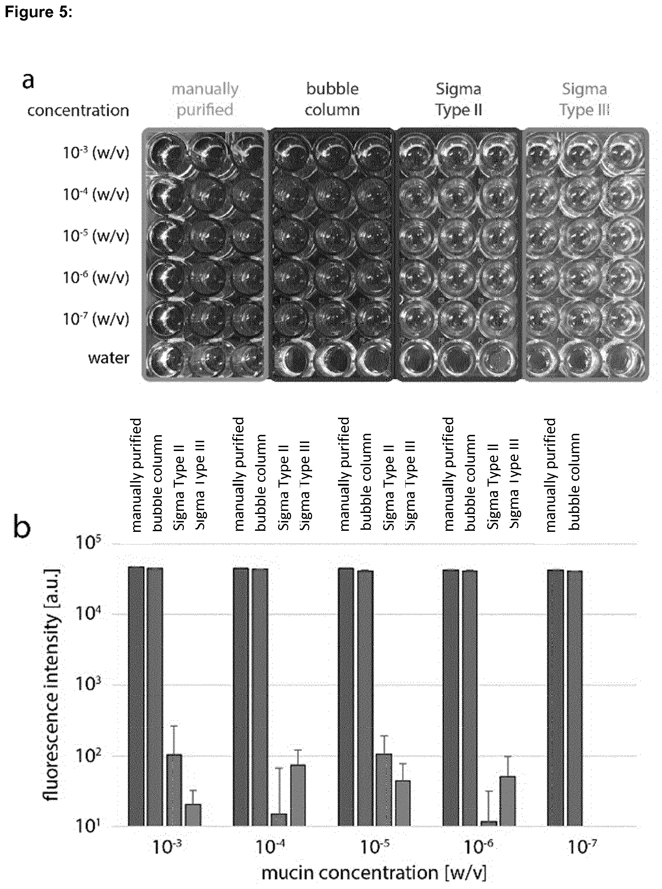

**Figure 5** shows the results of an enzyme-linked immunosorbent assay (ELISA) test for mucin detection. A mucin-specific antibody (Anti-MUC5AC) is used to detect mucin in a test solution containing mucin manually purified according to Schömig et al., a commercially available PGM (Sigma Type II and Type III) and a mucin purified according to the present invention, respectively. The anti-MUC5AC antibody exhibits a high sensitivity for manually purified mucin according to Schömig et al. as well as for mucin purified according to the present invention, whereas almost no signal for commercially available mucin can be detected.

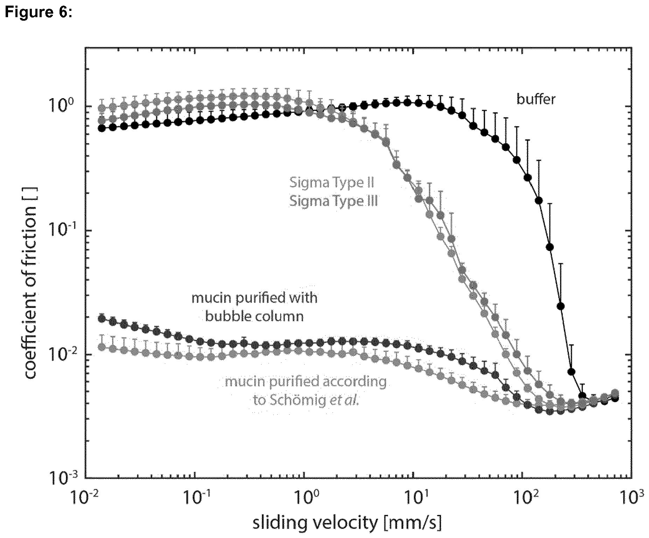

**Figure 6** shows the result of friction tests with commercially available PGM (Sigma Type II and Type III), mucin purified according to Schömig et al. and a mucin purified according to the present invention, respectively. The results show that mucin purified according to the present invention yields comparable friction coefficients as mucin purified according to Schömig et al. At low sliding velocities, the coefficient of friction is about 100 times lower than that obtained for commercially available mucins.

### DETAILED DESCRIPTION OF THE INVENTION

As explained above, the invention is directed to a method of isolating mucin from a mucin containing tissue. This method comprises contacting the mucin containing tissue with an aqueous solution in an aeriated bubble column under conditions suitable for transferring mucin into the aqueous solution, thereby producing a mucin containing solution.

It has been surprisingly found in the present invention that using an aeriated bubble column as described herein leads to a simple and effective isolation of mucin from a mucin containing tissue. The method is thus more time efficient and less susceptible to errors in comparison to the manual method of Schömig et al., *supra.* For example, the manual mucus harvesting step including opening the stomach, removing food residues and the manual mucus scraping from the opened stomach wall is no longer necessary. In addition, the overnight homogenization and the centrifugation steps previous to the ultracentrifugation are also no longer necessary. Further, the method of the present invention reduces complicated logistics and the storage of intermediate products drastically. For these reasons, the present invention provides a better reproducibility of mucin isolation, which leads to a more constant mucin quality. In addition, the process of the invention is easy scalable thus allowing production on an industrial scale. Further, the present invention is applicable to the isolation of any mucin and thus provides for a time efficient and effective isolation of any mucin secreted by mucous membranes. The method is suitable for the isolation and purification of any secreted mucins such as MUC2, MUC5AC, MUC5B, MUC6 und MUC7.

The invention will be further illustrated by the following explanations and examples.

In the present invention, the conditions suitable for transferring mucin into the aqueous solution may comprise any experimental condition suitable for facilitating the transfer of the mucin into this aqueous solution. Mucin is water soluble, thus the suitable conditions for a facilitated mucin transfer into the aqueous solution may be determined by the temperature, pH or the salt concentration of the solution. Further, the time over which the mucin containing tissue is contacted with the aqueous solution may play a role.

In the present invention, any mucin containing tissue can be used. Accordingly, the tissue may, for example, be of plant origin. For example, the mucin containing tissue may be a whole plant or parts thereof. An example for such a plant is a carnivorous plant such as a flypaper trap like *Pinguicula conzatti* or a great sundrew like *Drosera anglica,* which use mucilage secreted on the leaves to catch prey. Alternatively, the mucin containing tissue may be of animal origin. The tissue may, for example, be obtained from a mammal such as a farm animal including, but not limited to, a pig, a cow, a sheep, a horse, a goat, a donkey, a dog, a cat, a rabbit or poultry such as a bird, a chicken, a turkey, a duck or a goose. When obtained from poultry, the mucin containing tissue may also be the egg albumen. When obtained from a quadruped such as a pig or a cow the mucin containing tissue may be an organ or a part thereof. Thus, the tissue may be a mouth tissue comprising salivary glands, a tongue, an esophagus, a stomach, an intestine, a lung or a mixture thereof. Mucin containing tissue may also be a specific tissue of an animal organ such as the gastric tissue from a pig or a submaxillary gland from a cow. In another example of mucin isolation from an animal, the mucin containing tissue may be from a marine mollusk such as a clam or a cockle. The mucin containing tissue may also be obtained from a fish. Examples for suitable fish include eel or hagfish. When such fish are used as mucin source, the mucin containing tissue of an eel or hagfish may be its skin. The mucin containing tissue may also be sourced from a cnidarian such as a box jellyfish or a coral.

Turning to the conditions for the transfer of the mucin into the aqueous solution in more detail, the tissue may be present in any suitable processable form. The tissue may be used as a whole (for example, if skin or an intestine from a small animal such as a bird or tissue from a plant is used). In such a case, the mucin containing tissue may be everted to facilitate the accessibility of the aqueous solution to the mucin containing tissue in the bubble column. Alternatively, also pieces of the mucin containing tissue may be used. Consequently, the mucin containing tissue may then need to get cut before it is contacted with the aqueous solution. The cutting may be performed using any suitable method, for example manually or with a cutting device. The mucin containing tissue used may be pieces of organ such as tongue pieces, esophagus pieces, stomach pieces, intestine pieces, lung pieces, skin pieces or a mixture thereof, to name only a few possibilities. The tissue pieces may have any shape, for example an essentially rectangular, square or circular shape. For example, if stomach pieces with an essentially rectangular shape are used, these pieces have a longest dimension of about 1cm to about 20cm, or of about 4cm to 15cm. Using (relatively) small tissue pieces may have the advantage that the aqueous solution is able to access the complete mucous surface of the mucin containing tissue. This may contribute to the effective mucin isolation of the present invention. However, the dimensions of mucin containing tissue may also be greater, for example, if a whole organ is used. If, as an illustrative example, the skin of a whole hagfish is used, the tissue (skin) may be about 30cm to about 90 cm in the longest dimension. It is within the knowledge of the person of average skill in the art to choose an experimental setup, comprising the size of the bubble column, the gas flow and the flow regime, that ensures that also mucin can be isolated from larger tissue with the same effectiveness as the mucin isolation from smaller tissue pieces.

In the present invention, any solution can be used that is suitable for transferring mucin from a mucin containing tissue into the solution. The solution may have a certain osmolarity since a high salt concentration decreases the Debye screening length causing attenuated electrostatic interactions of the charged mucin. Thus, a certain salt content in the aqueous solution facilitates the detachment of mucin from the mucin containing tissue consequently contributing to the liquefaction of the mucus. Further, the salt content of the solution may also influence the quality of the mucin product since impurities such as charged molecules may detach from the mucin structure due to the attenuated electrostatic interactions of the mucin. Thus, the aqueous solution suitable for the present invention may have an osmolarity of about 350mosm/L. The aqueous solution of the present invention may also have a higher osmolarity, for example, an osmolarity of up to about 400mosm/L, of up to about 500mosm/L, of up to about 600mosm/L, of up to about 700mosm/L or of up to about 1000mosm/L. Accordingly, the aqueous solution suitable for the present invention may be a saline solution or a saline buffer. An illustrative example for a suitable saline solution is a physiological solution, which may be prepared with a salt comprising of monovalent cations. An illustrative example for such a salt may be any commonly used salt such as sodium chloride or potassium chloride in which sodium or potassium is the monovalent cation. The saline solution may also be prepared with commonly used buffering salts such as disodium hydrogen phosphate, potassium dihydrogen orthophosphate or monopotassium phosphate. Typically, any aqueous saline buffer that is able to buffer within a range of about pH 4 to about pH 9 can be used in the present invention. Illustrative examples include, but are not limited to PBS, tris-buffered saline (TBS), Veronal-buffer, citrate-phosphate buffer, tris(hydroxymethyl)aminomethane (TRIS), TE buffer, 4-(2-hydroxyethyl)-1-piperazineethanesulfonic acid (HEPES) buffer, Ringer's solution or a bicarbonate buffer system. In one illustrative embodiment of the method, the aqueous solution is PBS such as 1x, 2x or 3x PBS.

As already described above, in the present invention the mucin containing tissue is contacted with the aqueous solution in the bubble column to transfer the mucin into the aqueous solution. Contacting the tissue with the solution typically comprises moistening of the entire mucin containing tissue with the aqueous solution. This way, the solution may access the complete surface of the mucin containing tissue, thus facilitating the transfer of mucin into the solution. In other words, the mucin containing solution is contacted with a suitable amount of the aqueous solution to allow the transfer of mucin into the aqueous solution. The suitable amount of aqueous solution may be at least half the volume of the tissue containing solution. In one example, the suitable amount of aqueous solution may be a 1:1 mixture (v/v) of mucin containing tissue to saline buffer, wherein the mixture may be prepared by contacting one volume mucin containing tissue to one volume of the saline buffer in the bubble column. Further, a 1:2 mixture (v/v), or a 1:3 mixture (v/v), or a 1:4 mixture (v/v), or a 1:5 mixture (v/v) prepared accordingly may be also suitable for the present invention. During the contacting step in the bubble column, impurities such as food residues, gastric fluids or cell lysates may decrease the pH to a level suitable for the activity of mucin degrading enzymes. The enzyme pepsin, which is found in the gastric juice and which is active in a range of pH 2 to pH 4, may be an example for such a mucin degrading enzyme. Thus, it may be advantageous to adjust the pH of the aqueous solution during the contacting process in the bubble column. In the present invention, the pH may be set in a range of about pH 4 to about pH 9 during the step of contacting the mucin containing tissue with the aqueous solution. The pH may, for example, be set to be about 7, or about 8 or to be in the pH range of about 7 to about 8. For pH adjustment and/or the pH maintenance during the contacting process, a saline buffer may be used.

In the context of contacting mucin containing tissue with the aqueous solution in the bubble column, any suitable period of time allowing the transfer of the mucin into the aqueous solution can be applied. The mucin containing tissue may, for example, be contacted with the aqueous solution in the bubble column e.g. for as short as about 10min or at least about 30min. In one example, the mucin containing tissue may be contacted with the aqueous solution over a longer period e.g. for at least about 1h, about 2h, about 3h, about 4h, about 5h, about 6h, about 10h, about 12h or even about one or two days. To inhibit bacterial growth and thus bacterial degradation of the mucin during this contacting process, the temperature of the aqueous solution may be adjusted. For example, the temperature of the aqueous solution may be in the range of about 0°C to about 42°C, in the range of about 2°C to about 6°C, or in the range of about 3°C to 5°C. Further, a bactericidal or bacteriostatic agent may be added to the aqueous solution in the bubble column. Any bactericidal or bacteriostatic agent suitable to avoid or inhibit bacterial growth in an aqueous solution can be used. An illustrative example for a suitable bactericide may be sodium azide or merthiolate. Examples for a suitable bacteriostatic agent include but are not limited to chloramphenicol, macrolides, novobiocin, spectinomycin, sulfonamides and tetracyclines.

After contacting the mucin containing tissue with the aqueous solution, the liquid phase containing the mucin may be separated from the solid tissue. For this purpose, any separation means suitable for restraining the solid tissue and/or coarse impurities such as food residues from the liquid phase can be used. For example, the tissue may be separated by decanting, straining, sieving, filtrating or by the use of a disc separator. Afterwards, the separated mucin containing solution may be subjected to further purification. Thereby, the mucin is purified from impurities such as cellular components. In one example, the mucin containing solution may be contacted with a chemical for a certain period of time to ensure a binding or denaturation of the impurities. In a following step, this chemical containing mucin solution may be centrifuged so that the impurities can be removed in form of a pellet. For this purpose, any chemical suitable for denaturizing proteins in a physiological solution can be used. Examples for such suitable chemicals are urea or guanidine hydrochloride. In one example, the mucin containing solution may be contacted with about 4M, or about 5M, or about 6M, or about 7M, or about 8M urea for about 10 min to about 24 h, for example for about 3 h to bind cellular impurities. Thus, the impurities may precipitate allowing their removal by centrifugation leaving the mucin in the supernatant. In an alternative example for purifying mucin from cellular impurities, the mucin containing solution may be subjected to ultracentrifugation leaving the purified mucin in the supernatant. When ultracentrifugation is used, the mucin containing solution may be centrifuged at about 5000 x g to about 250000 × g over a period of about 30min to about 30h. Thus, in one example, the mucin containing solution may be subjected to an ultracentrifugation at about 5000 × g over a period of about 30h. In other examples, the mucin containing solution may be subjected to an ultracentrifugation at about 250000 × g over a period of about 36min or at about 150000 × g over a period of about 60min. In another alternative example for purifying mucin from cellular impurities, the mucin containing solution is subjected to filtration. For this purpose, any filter suitable for letting mucin pass and retaining cellular impurities can be applied. An example for such a suitable filter may be a paper filter, a membrane filter, a glass fiber filter, a syringe filter, a filter sleeve or a sterile filter unit. In a purely illustrative example, a commercially available tea or coffee filter may also be used. The person skilled in the art will know how to select a suitable filter for purifying mucin from cellular impurities.

The mucin may be further purified in the present method to remove lipids, macromolecules such as polymers or proteins or previously added compounds such as bactericidal or bacteriostatic agents. For this reason, the mucin containing solution may be subjected to a size exclusion chromatography (SEC) after ultracentrifugation. The SEC may be carried out using a saline solution or saline buffer. The salt content in the solution or buffer decreases the electrostatic interactions of the mucin molecule, thereby improving the purification and the quality of the mucin. Any saline solution or saline buffer with high salt content can be used for the SEC step of the present invention. As an illustrative example, the ultracentrifugated mucin containing solution is subjected to a SEC with a saline buffer, wherein the saline buffer is preferably 1x, 2x or 3x PBS. After the SEC, the mucin containing solution may be subjected to a diafiltration to concentrate and further purify the mucin. Also, the diafiltration may be performed with a saline solution or saline buffer. For example, the salt concentration of the saline solution or saline buffer used for the diafiltration may have a salt concentration in the range of about 200mM to about 5M, or in the range of about 1.5M to about 2.5M. The present method may also comprise a desalination of the diafiltrated mucin containing solution using distilled water or ultrapure water. By desalinating the mucin containing solution, quality fluctuations may be avoided since the electrostatic interactions of the mucins of the present invention are reduced to an equally low conductivity level in every batch. For example, the salt concentration of the diafiltrated mucin containing solution is reduced to a conductivity of at least <50 µS/cm, more preferably <25 µS/cm, most preferably <10µS/cm. Finally, the purified mucin solution may be frozen at -80°C for long-term storage. Alternatively, the mucin may be dried for long-term storage. In this context, any drying procedure available to a person skilled in the art may be used. For example, the purified mucin may be freeze-dried, lyophilized or spray-dried.

The present disclosure, which is not part of the invention, further describes a mucin obtained by the method described herein. The mucin obtained by this method may have lubricious properties, which are comparable to the properties of the manually isolated mucin of Schömig et al., *supra.* Thus, the present disclosure describes a mucin, which may be structurally undamaged or less damaged than the commercially available mucin products. The structural integrity of the mucin molecule isolated according to the present invention may be detectable using antibodies in an enzyme-linked immunosorbent assay (ELISA). Therefore, the mucin obtained by the method of the present invention may be more suitable for use in a (molecular) biological or medical application and/or for use in a medical product or compound. For example, the mucin obtained by the method of the present invention may be a biological lubricant more suitable for use in a medical product or more suitable for use on a medical device. Without wishing to being bound by theory, it also may be that the mucin obtained by the method of the present invention is more suitable for use as a barrier for invading pathogens. The present disclosure, which not part of the invention, also describes a composition comprising the mucin obtained by the method of the present invention. This composition may be for use in a medical product, e.g. as an additive in a mouth or nose spray.

The present invention also includes the use of a bubble column for isolating mucin from a mucin containing tissue. Accordingly, any bubble column suitable for aerating and thus mixing the mucin containing tissue in the aqueous solution can be used. Such a bubble column consequently facilitates the transfer of the mucin into the solution. The bubble column suitable for the present invention may comprise components depicted in **Figure 3****,** i.e. a hollow chamber (1), which may be sealed to the outside, a gas inlet (2), a non-return valve (3), a gas outlet (4), an external cooling system (5) and a control system for pH and a control system for the conductivity of the liquid. The gas inlet (2) of the bubble column may comprise several nozzles on the bubble column bottom. The gas outlet (4) of the bubble column may be mounted on the top or the upper side of the hollow chamber (1) of the bubble column. In one embodiment, the bubble column is optionally equipped with a filter and a return system to prevent foam from leaking and to return the liquid to the system. The bubble column can be made from any suitable material. The bubble column may be of poly methyl methacrylate (PMMA), stainless steel or glass, to mention only a few suitable materials. Likewise, the bubble column may have any suitable dimensions, depending on the intended capacity, for example. As an illustrative example, the bubble column may have at least an inner diameter of about 100mm to about 250mm and a height of at least about 150mm to about 750mm. Thus, the bubble column may have a volume of about 1 liter to about 37 liters. Consequently, in one illustrative example the bubble column may have at least an inner diameter of about 190mm and at least a height of about 500mm resulting in a volume of about 14 liters. In the present invention, the bubble column may contain an aqueous solution such as a saline solution or a saline buffer to isolate mucin from a mucin containing tissue. For the aeriation of the aqueous solution any gas may be used, wherein the gas may be preferably air or an inert gas or a mixture thereof. An example for an inert gas may be nitrogen. However, while economically not preferred, also a noble gas such as helium, neon, argon, krypton, xenon, or radon may be used as inert gas. The gas flow generates bubbles in the aqueous solution, which cause shear and flow forces on the mucous surface of the mucin containing tissue. Thus, the bubbles may contribute to transferring mucin into the aqueous solution.

The present invention provides an easy and time efficient method for mucin isolation, which also achieves a higher mucin yield compared to the manual mucin isolation of Schömig et al., *supra.* Thus, when pig stomachs are used, the mucin yield is increased from about 65mg per stomach to about 200mg per stomach by the present invention.

The invention will be further illustrated by the following non-limiting Experimental Examples.

### EXPERIMENTAL EXAMPLES

### Example 1: PGM isolation by using an aeriated bubble column

### Materials:

*Body parts of the bubble column (custom-made from PMMA)*
- Cylinder (*H* = 500 mm, *d*ᵢ = 190 mm, *d*ₐ = 200 mm) with two flanges (containing 6 regularly orientated M5 threads each) to attach bottom and top lid
- Bottom plate containing 5 regularly orientated holes (d = 3 mm) for gas supply
- Bottom lid with a central G1/4 thread for gas supply
- Top lid with central G1/4 thread as a gas exit
- Lid for gas overflow filter
- Overflow connector with thread for 0.5 L flask and overflow lid (both made from stainless steel)

*Labware for assembling the bubble column*
- 0.5 L flask for overflow filter
- Pneumatic tubings for gas supply (*d*ᵢ = 6 mm, *d*ₐ = 8 mm)
- Manometer (0 - 10 bar)
- Gas valve
- O-rings
- Steel grid (1 cm grid size)
- M5 bolts
- Pneumatic connector (with thread)
- Pneumatic clutches
- Non-return valve
- 10 L beakers

*Labware for Mucin extraction:*
- beakers, 1 × 2 L (plastic), several 5 L
- garbage bags for the stomachs (~50 L)
- pH meter
- Pipetboy + serological pipettes, 50 ml
- 1x Schott flask 2 L
- bottletop dispenser
- centrifuge tubes, 50 mL (∼100 per 20 stomachs)

*Labware for Centrifugation and SEC:*
- Beckmann Optima LE70 ultra centrifuge
- 6x 70 mL Beckmann centrifuge tubes for ultra-centrifugation
- GE Healthcare ÄKTA purifier system
- XK50/100 column packed with Sepharose 6FF
- glass cylinder, 250 mL
- Schott flasks, 250 mL, 1 L, 2 L, several 5 L
- (reuse) Falcon centrifuge tubes, 50 mL (8 per SEC run)

*Labware for Crossflow filtration:*
- Schott flasks, 1x 5 L, 1x 2 L
- beakers 2× 0.5 L, 5 L
- conductometer
- graduated cylinder, 250 mL
- Eppendorf tubes, 5 mL (∼20 per run)
- centrifugation tubes, 50 mL (∼3 per run)
- dispenser pipette + 50 mL combitip *Consumables and chemicals*
- 2-propanol
- Milli-Q water
- Paper towels
- 1xPBS
- 3x PBS (contains 18.36 mM Na₂HPO₄, 11.64 mM NaH₂PO₄ and 510 mM NaCl, pH = 8)

### Assembly of the bubble column

All parts of the bubble column were cleaned with 2-propanol. Afterwards, an O-ring was placed in the bottom flange of the main cylinder before the bottom plate was placed onto the flange. A second O-ring was placed in the bottom lid, before the bottom lid was placed onto the bottom plate. Six M5 bolts were used to screw the bottom lid, bottom plate and main cylinder together. Then, an O-ring was placed in the top flange of the main cylinder. Afterwards, a pneumatic connector was installed to the central thread in the bottom lid. Pneumatic tubing with a length of about 20cm connected the pneumatic connector with a non-return valve. The non-return valve was also connected to a closed gas valve via another pneumatic tubing of 20cm length. A pneumatic tubing was also used to connect the gas valve to a manometer and to a pneumatic clutch, which was later used as a coupling piece for the gas supply. Another pneumatic connector was installed on the central thread in the top lid, before it was connected to an overflow connector placed on a 0.5 L flask via a pneumatic tubing. Upon the overflow connector, a piece of paper towel was placed before the overflow lid was fixed with four M5 bolts.

### Operation of the bubble column

The bubble column was operated at 4°C. Thus, the assembled bubble column was placed in a cold room and then connected to compressed air supply (oil free, 10 bar) via the pneumatic clutch. Four stomachs of freshly slaughtered pigs (about 2 liters porcine stomach) were cut into 6 pieces and put into the bubble column. Then, 4 liters of pre-cooled (4°C) 3xPBS was added to the porcine stomachs in the bubble column (i.e. one volume of mucin containing tissue is contacted with twice the volume of the saline buffer in the bubble column). After the top lid of the bubble column was fixed with six M5 bolts and connected to the overflow flask via pneumatic tubing, the gas flow was started carefully. The gas pressure was slowly adjusted to 3 bar using the manometer. Stomach pieces and buffer were mixed for about 3h before the gas supply was stopped. The gas supply was dismounted behind the non-return valve to avoid fluid from leaking. The top lid was dismounted and the contents of the bubble column comprising the porcine stomachs and the mucin containing solution was decanted into a 10 L beaker using a grid. The mucin containing solution was then subjected to ultracentrifugation. If necessary, the mucin containing solution can be stored at -80°C until further use. The bubble column was then disassembled and cleaned with Milli-Q water and 2-propanol.

### Ultracentrifugation of the mucin containing solution

In a first step, 70ml centrifuge tubes (Beckmann) were filled up to 90% with the decanted mucin containing solution. After taring, the closed centrifuge tubes were carefully inserted into a Ti-45 rotor, and the rotor was inserted into the ultracentrifuge without scratching the coding disk at the bottom of the rotor. The mucin containing solution was ultracentrifuged at 4°C and 45000rpm (Ti-45)/150000 x g for about 1h. Afterwards, the supernatant of all centrifuged tubes was decanted into one 2I Schott flask. All tubes and also the 2I Schott flask were kept at 4°C when not in direct use. The collected and pooled supernatant of all runs can be divided into 50ml Falcon tubes and stored at -80°C for further use. The ultracentrifuge was cleaned manually. An alternative for this step is a filtration using paper filters as described as first step for the SEC.

### SEC of the mucin containing solution

First, the supernatant collected after the ultracentrifugation was poured through a paper filter (commercially available tea filter) into a 250ml glass cylinder and stored at 4°C. Then, an ÄKTA purifier system was prepared for SEC by purging the sample inlet to be used with 50ml 1× PBS. 50ml Falcon tubes were placed into the autosampler and the fractionation volume was adjusted to 44ml per falcon tube. A 5I bottle of 1xPBS and the 250ml glass cylinder containing the mucin containing solution were connected with the ÄKTA purifier system using inlet tubes. After the waste bottle was emptied the purification was started manually. After about 90min the automatically collected samples were harvested. At this point the collected samples can be also stored at -80°C for further use. The ÄKTA purifier system was cleaned after the run was completed.

### Crossflow-Filtration

The samples of 3 ÄKTA runs were thawed overnight at 4°C (alternatively in a water bath at room temperature on the day of crossflow filtration). The mucin containing solution was filled into the reservoir of the clean crossflow filtration device. Then, the osmolarity of the solution was adjusted by adding sodium chloride (NaCl) until a NaCl concentration of 2M is reached. Until all NaCl was dissolved, the pump was operated at low speed (max. 200rpm). Afterwards, the pump was operated at 400rpm and about 10 psi retentate pressure until the mucin solution was concentrated to a volume of about 300ml. By using the Rinsing/Diafiltration mode, the mucin solution was dialyzed against ultrapure water until a conductivity of ≤ 10 µS/cm was reached (400 rpm and ~ 10 psi retentate pressure). Then, the mucin solution was concentrated to a volume of about 70ml to 90ml. The concentrated mucin solution was aliquoted in labelled 5ml Eppendorf tubes (4.5ml per aliquot) and stored at - 80°C.

### Lyophilization

The purified mucin solution was freeze dried. For this, holes were drilled into the lids of the still frozen 5ml Eppendorf tubes. Then, these prepared frozen Eppendorf tubes were put into the vacuum chamber. After 2 to 3 days the mucin was freeze-dried and ready to be harvested.

After lyophilization, a mucin yield of about 200mg per porcine stomach was determined. This is a significant improvement compared to the manual mucin isolation according to Schömig et al. which yields only 65mg per stomach. Thus, the present invention provides a significant improvement for mucin isolation since the method described herein provides for a time efficient, effective and scalable isolation of mucin while at the same time yields about three times more mucin than the prior art with constant quality (as shown in the following Examples 2 and 3).

### Example 2: Experiment showing that the mucin isolated according to the present invention is detectable by mucin-specific antibodies in contrast to commercially available mucin

To test the mucin (MUC5AC) content in an aqueous solution, indirect enzyme linked immunosorbent assays (ELISA) were performed. Therefore, a solution containing 0.00001-0.1 % (v/w) mucin was pipetted into wells of a 96 well cell culture plate and incubated for 1 h to allow the mucin to adsorb to the surface of the wells. Afterwards, all samples were removed, and the wells were filled with a blocking buffer (5 % milk powder dissolved in PBS-Tween, i.e. PBS containing 1 mg/mL Tween 20, Carl Roth, pH = 7.4) and incubated at 4 °C overnight.

On the next day, the blocked wells were rinsed with PBS-Tween for three times. Afterwards, the samples were incubated with a primary antibody (200 µL per well) for 60 min while being placed on a shaker (Promax 1020, Heidolph Instruments GmbH & Co. KG, Schwabach, Germany). For this step, specific antibodies for MUC5AC detection (ABIN966608, antibodies-online GmbH, Aachen, Germany) were used. The recognition sequence of these mucin antibodies is located in the non-glycosylated C-terminus of the mucin molecule. A damage or complete absence of this C-terminus or even damage in the glycosylation pattern may cause a conformational change of the molecule, which again may result in inaccessibility to the antibodies. Thus, the binding of antibodies to MUC5AC isolated according to the present invention is a qualitative feature for mucin integrity and thus for native mucin.

The mucin antibodies were diluted 1:400 in blocking buffer. After incubating, the samples were rinsed again with PBS-Tween. A second antibody staining was then performed using horse radish peroxidase (HRP) conjugated goat anti mouse (murine) IgG antibodies (ABIN237501, antibodies-online GmbH). These secondary antibodies were diluted 1:5000 in blocking buffer. Incubation was allowed to take place for 120 min on a shaker at room temperature (RT). Afterwards, the samples were washed in pure PBS (since Tween tends to interfere with the solutions used for following steps). After washing the samples, 100 µL of QuantaRed Working Solution consisting of 50 parts QuantaRed Enhancer Solution, 50 parts QuantaRed Stable Peroxide and one part of QuantaRed ADHP Concentrate (QuantaRed Enhanced Chemifluorescent HRP Substrate Kit 15159, Thermo Fisher Scientific, Waltham, Massachusetts, USA) were added to each well. Since the Working Solution is light sensitive, direct light contact was avoided.

After 30 min of incubation at RT, the peroxidase activity was stopped by adding 20 µL of QuantaRed Stop Solution to each well. The fluorescence signal of the converted substrate was measured with a multi-label plate reader (Viktor3, PerkinElmer, Inc., Massachusetts, USA). Fluorescence was measured at a wavelength of 570 nm using a data acquisition time of 0.1 s.

### Example 3: Friction tests showing the lubrication properties of mucin isolated and purified according to the present invention in comparison to commercial available mucin

For friction measurements, a commercial shear rheometer (MCR 302, Anton Paar, Graz, Austria) was equipped with a tribology unit (T-PTD 200, Anton Paar). The measurements were performed in a ball-on-cylinder geometry. As friction partners in the tribology setup, three PDMS cylinders (as described earlier) and steel spheres with a diameter of 12.7 mm (1.4301, S_{q} < 0.2 mm; Kugel Pompel, Vienna, Austria) were used. Measurements were performed at a constant normal load of *F*_{N} = 6 N. This normal load was chosen such that friction in the boundary, mixed, and hydrodynamic regimes could be probed within the accessible speed range. According to the Hertzian contact theory using the Young's moduli and Poisson's ratios of steel (*Eₛₜₑₑₗ* = 210 GPa, *vₛₜₑₑₗ* ≈ 0.30) and PDMS (*E_{PDMS}* = 2 MPa, *v_{PDMS}* ≈ 0.49), this normal load results in an average contact pressure *p*₀ of ∼0.31 MPa.

The speed-dependent friction behavior was evaluated by performing a logarithmic speed ramp from ~700 to 0.001 mm s⁻¹. The friction coefficient was measured at 48 distinct speed levels for 10 s each. Before the first measuring point, the system was allowed to stabilize at the highest rotational speed for 30 s. For each measurement, 600 µL of 0.1 % (w/v) solutions of the respective mucins (dissolved in 20 mM HEPES pH 7.0) were used as lubricants.

It will be readily apparent to a person skilled in the art that varying substitutions and modifications may be made to the invention disclosed herein.

All patents and publications mentioned in the specification are indicative of the levels of those of ordinary skill in the art to which the invention pertains.

The inventions illustratively described herein may suitably be practiced in the absence of any element or elements, limitation or limitations, not specifically disclosed herein. Thus, for example, the terms "comprising", "including", "containing", etc. shall be read expansively and without limitation. Additionally, the terms and expressions employed herein have been used as terms of description and not of limitation, and there is no intention in the use of such terms and expressions of excluding any equivalents of the features shown and described or portions thereof, but it is recognized that various modifications are possible within the scope of the invention claimed. Thus, it should be understood that although the present invention has been specifically disclosed by preferred embodiments and optional features, modification and variation of the inventions embodied herein disclosed may be resorted to by those skilled in the art, and that such modifications and variations are considered to be within the scope of this invention.

When used herein "consisting of" excludes any element, step or ingredient not specified in the claim element. When used herein, "consisting essentially of" does not exclude materials or steps that do not materially affect the basic and novel characteristics of the claim.

Further embodiments of the invention will become apparent from the following claims.

### REFERENCES

Lieleg, O., Lieleg, C., Bloom, J., Buck, C. B. & Ribbeck, K. Mucin biopolymers as broad-spectrum antiviral agents. Biomacromolecules 13,1724-1732 (2012).
Käsdorf, B. T. et al. Mucin-Inspired Lubrication on Hydrophobie Surfaces. Biomacromolecules 18,2454-2462 (2017).
Ma, L., Gaisinskaya-Kipnis, A., Kampf, N. & Klein, J. Origins of hydration lubrication. Nature Communications 6,6060 (2015).
Biegler, M., Delius, J., Käsdorf, B. T., Hofmann, T. & Lieleg, O. Cationic astringents alter the tribological and rheological properties of human saliva and salivary mucin solutions. Biotribology 6,12-20 (2016).
Winkeljann, B., Boettcher, K., Balzer, B. N. & Lieleg, O. Mucin Coatings Prevent Tissue Damage at the Cornea-Contact Lens Interface. Advanced Materials Interfaces 4, 1700186 (2017).
Boettcher, K., Winkeljann, B., Schmidt, T. A. & Lieleg, O. Quantification of cartilage wear morphologies in unidirectional sliding experiments: Influence of different macromolecular lubricants. Biotribology (2017).
Kocevar-Nared, J., Kristl, J., Smid-Korbar, J. Comparative rheological investigation of crude gastric mucin and natural gastric mucus. Biomaterials 18,677-681 (1997).
Schömig, V. J. et al. An optimized purification process for porcine gastric mucin with preservation of its native functional properties. RSC Advances 6,44932-44943 (2016).

## Claims

1. A method of isolating mucin from a mucin containing tissue comprising contacting the mucin containing tissue with an aqueous solution in an aeriated bubble column under conditions suitable for transferring mucin into the aqueous solution, thereby producing a mucin containing solution.

2. The method of claim 1, wherein the conditions suitable for transferring mucin into the aqueous solution comprises contacting the mucin containing tissue with the aqueous solution over a suitable period of time at a suitable temperature.

3. The method of claim 1 or 2, wherein the mucin containing tissue is of plant or animal origin.

4. The method of any one of claims 1 to 3, wherein the mucin containing tissue is a gastric tissue or comprises a submaxillary gland.

5. The method of claim 1 or 2, wherein the aqueous solution has an osmolarity of at least about 350 mosm/L.

6. The method of any one of claims 1 to 5, wherein the pH of the aqueous solution is in a range of 4 to 9 during the step of contacting the mucin containing tissue with the aqueous solution, preferably wherein the pH is approximately 7, more preferably wherein the pH is approximately 8.

7. The method of any one of claims 1 to 6, wherein the mucin containing tissue is contacted with the aqueous solution in the bubble column over a period of at least 10 min, preferably at least 30 min more preferably about 1 h.

8. The method of any one of claims 1 to 7, wherein the temperature of the aqueous solution is in the range of about 0°C to about 42°C, preferably in the range of about 2°C to about 6°, more preferably in the range of about 3°C to 5°C.

9. The method of any one of claims 1 to 8, wherein the mucin containing tissue is separated from the liquid phase after a suitable period of time by decanting, straining, sieving or filtrating.

10. The method of claim 9, wherein the separated mucin containing solution is subjected to ultracentrifugation or filtration.

11. The method of claim 10, wherein the ultracentrifugated or filtrated mucin containing solution is subjected to size exclusion chromatography.

12. The method of claim 11, wherein after the size exclusion chromatography the mucin containing solution is diafiltrated with a sodium chloride solution.

13. A use of a bubble column for isolating mucin from mucin containing tissue.

## Patentansprüche

1. Verfahren zur Isolierung von Mucin aus einem Mucin-haltigen Gewebe, umfassend das In-Kontakt-Bringen des Mucin-haltigen Gewebes mit einer wässrigen Lösung in einer belüfteten Blasensäule unter Bedingungen, die geeignet sind, Mucin in die wässrige Lösung zu überführen, wodurch eine Mucin-haltige Lösung hergestellt wird.

2. Verfahren nach Anspruch 1, wobei die Bedingungen, die für das Überführen von Mucin in die wässrige Lösung geeignet sind, das In-Kontakt-Bringen des Mucin-haltigen Gewebes mit der wässrigen Lösung über einen geeigneten Zeitraum bei einer geeigneten Temperatur umfassen.

3. Verfahren nach Anspruch 1 oder 2, wobei das Mucin-haltige Gewebe pflanzlichen oder tierischen Ursprungs ist.

4. Verfahren nach einem der Ansprüche 1 bis 3, wobei das Mucin-haltige Gewebe ein Magengewebe ist oder eine Unterkieferdrüse umfasst.

5. Verfahren nach Anspruch 1 oder 2, wobei die wässrige Lösung eine Osmolarität von mindestens etwa 350 mosm/L aufweist.

6. Verfahren nach einem der Ansprüche 1 bis 5, wobei der pH der wässrigen Lösung in einem Bereich von 4 bis 9 während des Schritts des In-Kontakt-Bringens des Mucin-haltigen Gewebes mit der wässrigen Lösung liegt, wobei der pH vorzugsweise ungefähr 7 ist, besonders bevorzugt, wobei der pH ungefähr 8 ist.

7. Verfahren nach einem der Ansprüche 1 bis 6, wobei das Mucin-haltige Gewebe mit der wässrigen Lösung in der Blasensäule über einen Zeitraum von mindestens 10 min, vorzugsweise mindestens 30 min, besonders bevorzugt etwa 1 h, in Kontakt gebracht wird.

8. Verfahren nach einem der Ansprüche 1 bis 7, wobei die Temperatur der wässrigen Lösung im Bereich von etwa 0°C bis etwa 42°C, bevorzugt im Bereich von etwa 2°C bis etwa 6°C, besonders bevorzugt im Bereich von etwa 3°C bis 5°C liegt.

9. Verfahren nach einem der Ansprüche 1 bis 8, wobei das Mucin-haltige Gewebe nach einer geeigneten Zeitspanne durch Dekantieren, Abseihen, Sieben oder Filtrieren von der flüssigen Phase getrennt wird.

10. Verfahren nach Anspruch 9, wobei die abgetrennte Mucin-haltige Lösung einer Ultrazentrifugation oder Filtration unterzogen wird.

11. Verfahren nach Anspruch 10, wobei die ultrazentrifugierte oder filtrierte Mucin-haltige Lösung einer Größenausschlusschromatographie unterzogen wird.

12. Verfahren nach Anspruch 11, wobei nach der Größenausschlusschromatographie die Mucin-haltige Lösung mit einer Natriumchloridlösung diafiltriert wird.

13. Verwendung einer Blasensäule zur Isolierung von Mucin aus Mucin-haltigem Gewebe.

## Revendications

1. Procédé d'isolement de mucine à partir d'un tissu contenant de la mucine, comprenant la mise en contact du tissu contenant de la mucine avec une solution aqueuse dans une colonne à bulles aérée dans des conditions appropriées pour transférer la mucine dans la solution aqueuse, produisant ainsi une solution contenant de la mucine.

2. Procédé selon la revendication 1, dans lequel les conditions appropriées pour transférer la mucine dans la solution aqueuse comprennent la mise en contact du tissu contenant de la mucine avec la solution aqueuse pendant une période de temps appropriée à une température appropriée.

3. Procédé selon la revendication 1 ou 2, dans lequel le tissu contenant de la mucine est d'origine végétale ou animale.

4. Procédé selon l'une quelconque des revendications 1 à 3, dans lequel le tissu contenant de la mucine est un tissu gastrique ou comprend une glande sous-maxillaire.

5. Procédé selon la revendication 1 ou 2, dans lequel la solution aqueuse a une osmolarité d'au moins environ 350 mosm/L.

6. Procédé selon l'une quelconque des revendications 1 à 5, dans lequel le pH de la solution aqueuse est dans une plage allant de 4 à 9 pendant l'étape de mise en contact du tissu contenant de la mucine avec la solution aqueuse, de préférence dans lequel le pH est d'environ 7, plus préférentiellement dans lequel le pH est d'environ 8.

7. Procédé selon l'une quelconque des revendications 1 à 6, dans lequel le tissu contenant de la mucine est mis en contact avec la solution aqueuse dans la colonne à bulles pendant une période d'au moins 10 min, de préférence d'au moins 30 min, plus préférentiellement d'environ 1 h.

8. Procédé selon l'une quelconque des revendications 1 à 7, dans lequel la température de la solution aqueuse est dans la plage allant d'environ 0 °C à environ 42 °C, de préférence dans la plage allant d'environ 2 °C à environ 6°, plus préférentiellement dans la plage allant d'environ 3 °C à 5 °C.

9. Procédé selon l'une quelconque des revendications 1 à 8, dans lequel le tissu contenant de la mucine est séparé de la phase liquide après une période de temps appropriée par décantation, égouttage, tamisage ou filtration.

10. Procédé selon la revendication 9, dans lequel la solution séparée contenant de la mucine est soumise à une ultracentrifugation ou à une filtration.

11. Procédé selon la revendication 10, dans lequel la solution ultracentrifugée ou filtrée contenant de la mucine est soumise à une chromatographie d'exclusion stérique.

12. Procédé selon la revendication 11, dans lequel, après la chromatographie d'exclusion stérique, la solution contenant de la mucine est diafiltrée avec une solution de chlorure de sodium.

13. Utilisation d'une colonne à bulles pour isoler de la mucine d'un tissu contenant de la mucine.
